# EUROPÄISCHE PATENTANMELDUNG

(11) **EP 3 967 334 A1**
(43) Veröffentlichungstag der Anmeldung: **16.03.2022**
(21) Anmeldenummer: 21193537.4
(22) Anmeldetag: 27.08.2021
(51) Int. Cl.: A61L 9/20, A47L 15/42, D06F 39/00, D06F 35/00, D06F 58/20, F25D 17/04

(54) **LUFTAUFBEREITUNGSVORRICHTUNG FÜR EIN HAUSHALTSGERÄT**

(30) Priorität: 10.09.2020 DE 102020123643; 10.09.2020 DE 102020123644; 20.08.2021 CN 202110960448
(71) Anmelder: emz-Hanauer GmbH & Co. KGaA, 92507 Nabburg (DE)
(72) Erfinder: Signorino, Manfredi, 92442 Wackersdorf (DE); Schemela, Benjamin, 92720 Schwarzenbach (DE); Ren, Shaojiang, Nanjing, 211122 (CN)
(74) Vertreter: Hannke Bittner & Partner

(57) **Zusammenfassung**

Die Erfindung betrifft eine Luftaufbereitungsvorrichtung (1) für ein Haushaltsgerät (100) mit einer Behältereinrichtung (102), wobei die Luftaufbereitungsvorrichtung (1) ein erstes Gehäuse (2) umfasst, in welchem zumindest eine Aufbereitungseinrichtung (3) angeordnet ist, wobei die Luftaufbereitungsvorrichtung (1) eine Zuleitungseinrichtung (4) und eine Ableitungseinrichtung (5) aufweist, wobei die Zuleitungseinrichtung (4) dafür vorgesehen und geeignet ist, einen Luftstrom (10) zu der Aufbereitungseinrichtung (3) zu leiten, wobei die Ableitungseinrichtung (5) dafür vorgesehen und geeignet ist, einen Luftstrom (10) von der Aufbereitungseinrichtung (3) weg zu leiten, wobei die zumindest eine Aufbereitungseinrichtung (3) einen Durchtrittskanal (6) für den Luftstrom (10), eine Strahlungsquelleneinrichtung (7) und eine Photokatalyseeinrichtung (8) umfasst.

## Beschreibung

Die Erfindung betrifft eine Luftaufbereitungsvorrichtung für ein Haushaltsgerät mit einer Behältereinrichtung. Weiterhin umfasst die Erfindung ein Haushaltsgerät mit einer Luftaufbereitungsvorrichtung.

Derartige Haushaltsgeräte können beispielsweise Geschirrspüler oder auch Waschmaschinen sein. Die Behältereinrichtung würde in einem solchen Fall ein Laugenbehälter sein, in dem sich die zu waschenden Gegenstände und die Waschflüssigkeit befinden. Ferner könnte ein solches Haushaltsgerät beispielsweise ein Kühlschrank oder Gefrierschrank sein, die Behältereinrichtung wäre demnach der Kühlraum, in welchem sich die zu kühlenden Gegenstände befinden.

Mit zunehmender Betriebsdauer ist es oftmals wünschenswert, dass die sich in der Behältereinrichtung befindliche Luft aufbereitet werden muss. In der Luft vorhandene Mikroorganismen - wie Viren, Bakterien, Hefen und Schimmelpilze - können die Gesundheit von Personen gefährden, Rohstoffe verunreinigen und Lebensmittel verderben. Weiterhin kann die Luft Gase, Flüssigkeiten und Feststoffe enthalten, welche einen unangenehmen Geruchseindruck hinterlassen. Zwar sind einige Haushaltsgeräte mit Einrichtungen versehen, welche diese Probleme beseitigen sollen, jedoch sind derartige Einrichtungen für jedes der Haushaltsgeräte speziell konstruiert. Es besteht somit ein Bedarf an einer Luftaufbereitungseinrichtung, welche kostengünstig herzustellen ist und für eine Vielzahl verschiedener Haushaltsgeräte einsetzbar ist.

Die Aufgabe der vorliegenden Erfindung besteht darin, eine Luftaufbereitungsvorrichtung für ein Haushaltsgerät bereitzustellen, welche die genannten Nachteile überwindet. Ferner ist es Aufgabe der Erfindung, ein Haushaltsgerät mit einer Luftaufbereitungsvorrichtung bereitzustellen.

Die Aufgabe wird gelöst durch die Gegenstände der Ansprüche 1 und 10. Die Unteransprüche umfassen bevorzugte Ausführungsformen.

Erfindungsgemäß wird eine Luftaufbereitungsvorrichtung für ein Haushaltsgerät mit einer Behältereinrichtung bereitgestellt, wobei die Luftaufbereitungsvorrichtung ein erstes Gehäuse umfasst, in welchem zumindest eine Aufbereitungseinrichtung angeordnet ist, wobei die Luftaufbereitungsvorrichtung eine Zuleitungseinrichtung und eine Ableitungseinrichtung aufweist, wobei die Zuleitungseinrichtung dafür vorgesehen und geeignet ist, einen Luftstrom bevorzugt aus der Behältereinrichtung zu der Aufbereitungseinrichtung zu leiten, wobei die Ableitungseinrichtung dafür vorgesehen und geeignet ist, einen Luftstrom der Aufbereitungseinrichtung weg bevorzugt zu der Behältereinrichtung zu leiten, wobei die zumindest eine Aufbereitungseinrichtung einen Durchtrittskanal für den Luftstrom, eine Strahlungsquelleneinrichtung und eine Photokatalyseeinrichtung umfasst.

Die Luftaufbereitungsvorrichtung umfasst demnach ein erstes Gehäuse, an welchem sowohl eine Zuleitungseinrichtung als auch eine Ableitungseinrichtung für den Luftstrom aus der Behältereinrichtung beziehungsweise in die Behältereinrichtung vorgesehen ist. Vorteilhafterweise sind alle kostspieligen Komponenten der Luftaufbereitungsvorrichtung in Form der Aufbereitungseinrichtung in dem ersten Gehäuse angeordnet. Vorteilhafterweise umfasst das Haushaltsgerät ein Gehäuse, in welchem die Behältereinrichtung angeordnet ist. Das erste Gehäuse der Luftaufbereitungsvorrichtung kann innerhalb oder außerhalb des Gehäuses des Haushaltsgeräts angeordnet sein. Durch die kompakte Ausführung der Luftaufbereitungsvorrichtung in einem separaten Gehäuse mit einer Zuleitungseinrichtung als auch einer Ableitungseinrichtung stellt die vorliegende Luftaufbereitungsvorrichtung ein modulares Element dar, welches in einer Vielzahl von Haushaltsgeräten implementiert, beziehungsweise verwendet werden kann. In dem separaten Gehäuse werden alle kostenintensiven Komponenten, in Form von der Aufbereitungseinrichtung konzentriert. Es wird somit eine modular verwendbare Luftaufbereitungsvorrichtung bereitgestellt, welche in einer Vielzahl von Haushaltsgeräten einsetzbar ist. Die kompakte Bauweise erlaubt die Implementierung an jeder verfügbaren Stelle in dem Gerät.

Durch den Luftstrom wird eine Teilmenge der Luft der Behältereinrichtung entnommen und durch die Aufbereitungseinrichtung aufbereitet. Die aufbereitete Teilmenge der Luft wird dann wieder über die Abgabeeinrichtung der Behältereinrichtung zugeführt. Durch einen vorteilhaften kontinuierlichen Betrieb kann die gesamte Luftmenge oder ein überwiegender Anteil der Luftmenge in der Behältereinrichtung aufbereitet werden. Unter einer Aufbereitung der Luft soll eine Reinigung und/oder eine Desinfektion und/oder ein Entfernen von Gerüchen verstanden werden.

Nach einer bevorzugten Ausführungsform emittiert die Strahlungsquelleneinrichtung elektromagnetische Strahlung. Bevorzugt ist zur Erzeugung einer photokatalytischen Reaktion die Photokatalyseeinrichtung mit zumindest einem Teil der elektromagnetischen Strahlung beaufschlagbar.

Nach einer bevorzugten Ausführungsform umfasst die Photokatalyseeinrichtung zumindest ein photokatalytisches Material. Vorzugsweise umfasst die Photokatalyseeinrichtung eine Photokatalyseoberfläche, welche zumindest ein photokatalytisches Material umfasst. Vorzugsweise ist das photokatalytische Material in der Photokatalyseoberfläche integriert. Bevorzugt besteht zumindest die Photokatalyseoberfläche zumindest teilweise aus zumindest einem photokatalytischen Material. Vorteilhafterweise umfasst die Photokatalyseoberfläche der Photokatalyseeinrichtung Abschnitte aus einem photokatalytischen Material. Bevorzugt besteht zumindest die Photokatalyseoberfläche der Photokatalyseeinrichtung gänzlich aus einem photokatalytischen Material. Bevorzugt wird die Photokatalyseoberfläche mit der elektromagnetischen Strahlung der Strahlungsquelleneinrichtung beaufschlagt.

Bevorzugt ist das photokatalytische Material der Photokatalyseeinrichtung ein Halbleiter. Halbleiter werden üblicherweise durch das sogenannte Bändermodell beschrieben und umfassen ein Leitungsband und ein Valenzband, welche durch die sogenannte Bandlücke energetisch voneinander getrennt sind. Die Größe der Bandlücke variiert mit dem entsprechenden individuellen Halbleitermaterial. Das Valenzband und das Leitungsband sind gemäß der Fermi-Verteilung mit Elektronen besetzt. Demnach ist am Temperaturnullpunkt das Valenzband besetzt und das Leitungsband unbesetzt. Ein einfallendes Photon kann ein Elektron-Loch-Paar erzeugen, falls die Energie des Photons größer oder gleich dem Energieunterschied in der Bandlücke ist. Durch die beweglichen Elektronen im Leitungsband kann vorteilhafterweise bei dem photokatalytischen Material eine chemische beziehungsweise photokatalytische Reaktion an der Oberfläche entstehen. Es wird dabei vorteilhafterweise ein Teilchen der Luft reduziert. Ein solches Teilchen kann vorzugsweise ein Molekül, ein Ion oder ein Atom sein. Durch diese Reduktion kann sich die chemische Eigenschaft bestimmter Bestandteile bereits ändern, so dass diese keinen unangenehmen Geruch mehr verursachen oder einfacher entfernbar, beispielsweise durch Abwaschen, sind. Weiterhin können durch die chemische beziehungsweise photokatalytische Reaktion freie Radikale entstehen. Freie Radikale sind Moleküle, Ionen oder Atome mit einem ungepaarten Elektron und sind hochgradig reaktiv. Diese freien Radikale können vorteilhafterweise mit den unerwünschten Mikroorganismen und Gasen der Luft reagieren und diese abtöten beziehungsweise in andere Gase umwandeln. Die Luft wird somit gereinigt von unerwünschten Mikroorganismen und unerwünschten Gasen, welche unangenehme Gerüche verursachen können. Nach einer bevorzugten Ausführungsform ist oder umfasst das photokatalytische Material Titan(IV)-oxid, Titandioxid, TiO₂. Denkbar wäre auch eine Beschichtung aus Titandioxid, welche auch einem anderweitigen Material beispielsweise einer Keramik aufgetragen ist.

Nach einer weiteren vorteilhaften Ausführungsform umfasst die Strahlungsquelleneinrichtung zumindest eine Strahlungsquelle. Vorzugsweise ist die zumindest eine Strahlungsquelle eine Leuchtdiode LED. Vorteilhafterweise emittiert die Strahlungsquelleneinrichtung elektromagnetische Strahlung mit einer Wellenlänge von kleiner als 400 nm. Vorteilhafterweise emittiert die Strahlungsquelleneinrichtung UV-Strahlung. Vorzugsweise ist die zumindest eine Strahlungsquelle eine UV-LED. Die emittierte elektromagnetische Strahlung, vorzugsweise in Form von UV-Strahlung, trifft auf die Photokatalyseeinrichtung beziehungsweise die Photokatalyseoberfläche und verursacht die oben beschriebene photokatalytische Reaktion. Bei einer vorteilhaften Verwendung von Titandioxid TiO₂ erfolgt bei einer Bestrahlung mit UV-Strahlung eine Erzeugung eines Elektron-Loch-Paares und die beschriebene chemische beziehungsweise photokatalytische Reaktion. Vorzugsweise kann Titandioxid natürliche und artifizielle Verunreinigungen in Luft und Wasser durch Bestrahlen mit UV-Strahlung entfernen, indem der Luftsauerstoff reduziert und die Verunreinigungen zu umweltfreundlichen Endprodukten oxidiert (mineralisiert) werden. Ferner kann vorteilhafterweise die Oberfläche von Titandioxid durch Absorption von UV-Strahlung superhydrophil werden. Vorteilhafterweise emittiert die Strahlungsquelleneinrichtung elektromagnetische Strahlung mit einer Wellenlänge in einem Bereich von 380nm bis 315nm. Derartige Strahlung wird auch als sogenannte UV-A Strahlung bezeichnet. Denkbar wäre jedoch auch die Verwendung von UV-B Strahlung (315nm - 280nm) und UV-C Strahlung (280nm - 100nm). Bei einer vorteilhaften Verwendung von UV-C Strahlung würde diese Strahlung bereits eine entsprechende Wirkung auf die Luft beziehungsweise den Luftstrom haben. Insbesondere die kurzwellige UV-Strahlung wirkt stark bakterizid. Sie wird von der DNA der Mikroorganismen absorbiert und zerstört dort ihre Struktur. Auf diese Weise werden die lebenden Zellen inaktiviert.

Nach einer weiteren vorteilhaften Ausführungsform ist die zumindest eine Strahlungsquelle auf einer Trägereinrichtung angeordnet. Vorteilhafterweise ist die Trägereinrichtung plattenartig ausgebildet. Bevorzugt ist die Trägereinrichtung als eine Leiterplatte oder Platine (PCB, printed circuit board) ausgebildet. Vorteilhafterweise ist auf der Trägerplatte weiterhin zumindest eine Treibereinrichtung für die zumindest eine Strahlungsquelle angeordnet. Bevorzugt ist die Trägereinrichtung im Wesentlichen gegenüberliegend von der Photokatalyseeinrichtung angeordnet. Vorteilhafterweise ist der Durchtrittskanal für die Luft beziehungsweise den Luftstrom zwischen der Trägereinrichtung und der Photokatalyseeinrichtung vorgesehen. Durch eine solche Anordnung kann eine optimale Bestrahlung der Photokatalyseoberfläche mit der elektromagnetischen Strahlung erfolgen. Ferner kann ein optimaler Kontakt der Photokatalyseoberfläche mit dem Luftstrom sichergestellt werden.

Nach einer weiteren vorteilhaften Ausführungsform ist zumindest eine Luftströmungseinrichtung vorgesehen, mittels welcher der Luftstrom in und/oder aus der Luftaufbereitungsvorrichtung generierbar ist. Dabei ist es von Vorteil, dass die Zuleitungseinrichtung eine Lufteintrittsöffnung aufweist und die Aufbereitungseinrichtung eine Luftaustrittsöffnung aufweist. Vorzugsweise erzeugt die zumindest eine Luftströmungseinrichtung an der zumindest einen Lufteintrittsöffnung einen Unterdruck. Durch diesen Unterdruck ist der Luftstrom in die zumindest eine Lufteintrittsöffnung erzeugbar. Vorzugsweise strömt der Luftstrom durch die zumindest eine Lufteintrittsöffnung über die Zuleitungseinrichtung in das erste Gehäuse der Luftaufbereitungsvorrichtung und aus dem ersten Gehäuse der Luftaufbereitungsvorrichtung über die Ableitungseinrichtung und durch die zumindest eine Luftaustrittsöffnung in die Behältereinrichtung. In dem ersten Gehäuse Luftaufbereitungsvorrichtung strömt der Luftstrom durch den Durchtrittskanal der Aufbereitungseinrichtung. Die zumindest eine Luftströmungseinrichtung befördert vorzugsweise den Luftstrom zu der Aufbereitungseinrichtung, durch diese hindurch und dann durch die zumindest eine Luftaustrittsöffnung wieder in die Behältereinrichtung.

Durch die Entnahme von einer bestimmten Luftmenge und die Zuführung einer bestimmten Luftmenge wird eine Luftzirkulation in der Behältereinrichtung erzeugt. Durch eine solche Luftzirkulation wird ein Großteil der Luft in der Behältereinrichtung, beziehungsweise im Wesentlichen die gesamte sich in der Behältereinrichtung befindliche Luft der Luftaufbereitungsvorrichtung sukzessive zugeführt.

Nach einer bevorzugten alternativen Ausführungsform wird der Luftstrom durch das Haushaltsgerät erzeugt und der Zuleitungseinrichtung der Luftaufbereitungsvorrichtung zugeführt.

Demnach kann die Luftaufbereitungsvorrichtung noch kompakter und ohne eine Luftströmungseinrichtung gebaut werden.

Nach einer bevorzugten Ausführungsform umfasst die Luftströmungseinrichtung eine Antriebseinrichtung, beispielsweise einen Motor, insbesondere einen Elektromotor, welcher einen Rotor beziehungsweise einen Ventilator antreibt, um den Luftstrom zu erzeugen. Nach einer weiteren vorteilhaften Ausführungsform ist die Luftströmungseinrichtung zumindest teilweise in das erste Gehäuse der Luftaufbereitungsvorrichtung integriert. Dabei könnte vorteilhafterweise der Rotor oder Ventilator zumindest teilweise oder gänzlich in dem ersten Gehäuse integriert sein. Dabei könnte auch die Antriebseinrichtung, beispielsweise ein Motor, in dem ersten Gehäuse der Luftaufbereitungsvorrichtung angeordnet sein. Hierdurch würde eine besonders kompakte Luftaufbereitungsvorrichtung bereitgestellt werden. Denkbar wäre jedoch auch, dass lediglich der Rotor oder Ventilator in dem ersten Gehäuse angeordnet ist. Die Antriebseinrichtung könnte dann außerhalb des ersten Gehäuses vorgesehen sein. Die notwendige Kraftübertagung von der Antriebseinrichtung zu dem Rotor könnte über eine Kraftübertragungseinrichtung erfolgen. Dies könnte eine Welle, ein Getriebe, eine Spindel oder eine ähnliche Einrichtung sein.

Nach einer weiteren bevorzugten Ausführungsform ist die Luftströmungseinrichtung an dem Gehäuse der Luftaufbereitungsvorrichtung angeordnet. Vorteilhafterweise umfasst die Luftströmungseinrichtung ein zweites Gehäuse, welches an dem ersten Gehäuse dichtend angeordnet ist. Dies kann durch eine kraft-, und/oder formschlüssige Verbindung erfolgen. Dies könnte beispielsweise eine Schnappverbindung, eine Schraubverbindung oder Ähnliches sein. Vorteilhafterweise ist die Verbindung lösbar. Dies hat den Vorteil, dass Komponenten ausgetauscht werden können und eine Reinigung der Gehäuse vorgenommen werden kann. Denkbar wäre auch eine stoffschlüssige Verbindung, beispielsweise eine Klebeverbindung oder eine Schweißverbindung. Vorzugsweise ist das zweite Gehäuse zwischen dem ersten Gehäuse der Luftaufbereitungsvorrichtung und der Zuleitungseinrichtung angeordnet. Durch eine derartige Anordnung kann der Luftstrom effektiv generiert werden.

Nach einer weiteren vorteilhaften Ausführungsform sind die Zuleitungseinrichtung und/oder die Ableitungseinrichtung schlauchartig ausgebildet. Vorteilhafterweise sind die Zuleitungseinrichtung und/oder die Ableitungseinrichtung aus einem flexiblen Material hergestellt. Durch ein solche vorteilhafte schlauchartige, flexible Zuleitungseinrichtung und/oder Ableitungseinrichtung wird ermöglicht, dass die Positionierung des ersten und gegebenenfalls des zweiten Gehäuses vereinfacht wird und auch auf eine einfache Weise veränderbar ist. Selbstverständlich können die Zuleitungseinrichtung und/oder die Ableitungseinrichtung auch aus formstabilen rohrartigen Elementen bestehen.

Nach einer weiteren vorteilhaften Ausführungsform ist eine Länge der Zuleitungseinrichtung und/oder der Ableitungseinrichtung derart ausgestaltet, dass bei einer Anordnung der Luftaufbereitungsvorrichtung entlang einer Höhenrichtung über einer Eintrittsöffnung und einer Austrittsöffnung der Behältereinrichtung ein Eintritt von einer Flüssigkeit in das erste Gehäuse weitestgehend unterbunden wird. Die Eintrittsöffnung der Behältereinrichtung für den Luftstrom ist dabei vorteilhafterweise mit der Luftstromaustrittsöffnung der Ableitungseinrichtung verbunden. Die Luftaustrittsöffnung der Behältereinrichtung ist bevorzugt mit der Lufteintrittsöffnung der Zuleitungseinrichtung verbunden. Durch die Anordnung der Luftaufbereitungseinrichtung, insbesondere des ersten Gehäuses, entlang der Höhenrichtung (Z) des Haushaltsgeräts über der Eintrittsöffnung und einer Austrittsöffnung der Behältereinrichtung wird somit verhindert, dass größere Mengen an Flüssigkeit in das erste Gehäuse beziehungsweise die Aufbereitungseinrichtung gelangen. Die Länge ist dabei derart bemessen, dass die unerwünschte Flüssigkeit durch die wirkende Gravitationskraft in ausreichender Weise daran gehindert wird in das erste und/oder das zweite Gehäuse zu gelangen. Vorteilhafterweise weisen die Zuleitungseinrichtung und/oder die Ableitungseinrichtung eine Länge von zumindest 10cm, bevorzugt zumindest 5cm auf.

Nach einer weiteren vorteilhaften Ausführungsform umfasst die Luftaufbereitungsvorrichtung eine Entfeuchtungseinrichtung. Der Luftstrom kann jedoch Flüssigkeitstropfen mit sich tragen, welche in unerwünschter Weise in das erste und/oder das zweite Gehäuse gelangen. Diese Feuchtigkeit kann durch die vorteilhafte Entfeuchtungseinrichtung entfernt werden. Bevorzugt ist die Entfeuchtungseinrichtung in dem ersten Gehäuse angeordnet. Vorteilhafterweise umfasst die Entfeuchtungseinrichtung eine Heizeinrichtung. Mittels der vorteilhaften Heizeinrichtung kann die Temperatur der Luft in dem Luftstrom erhöht werden. Die Luft kann demnach mehr Feuchtigkeit aufnehmen, welche durch den Luftstrom abtransportiert wird. Denkbar wären jedoch auch Entfeuchtungseinrichtungen, welche die Feuchtigkeit absorbieren, wie beispielsweise chemische Entfeuchtungseinrichtungen. Selbstverständlich können auch noch anderweitige Entfeuchtungseinrichtung verwendet werden.

Die vorliegende Aufgabe der Erfindung wird auch von einem Haushaltsgerät, umfassend zumindest einer Luftaufbereitungsvorrichtung nach einem der vorher beschriebenen Ausführungsbeispiele, gelöst. Das Haushaltsgerät kann dabei mit allen bereits obig im Rahmen der Luftaufbereitungsvorrichtung beschriebenen Merkmalen einzeln oder in Kombination miteinander ausgestattet sein und umgekehrt.

Ein solches Haushaltsgerät umfasst vorteilhafterweise ein Gehäuse und eine darin vorgesehene Behältereinrichtung. Vorzugsweise ist die Luftaufbereitungsvorrichtung außerhalb oder innerhalb des Gehäuses des Haushaltsgeräts anordenbar. Ein solches Haushaltsgerät kann vorteilhafterweise ein Geschirrspüler, eine Waschmaschine, ein Kühlschrank, oder ein anderweitiges Haushaltsgerät sein. Bei einem Haushaltsgerät in Form eines Geschirrspülers oder einer Waschmaschine wird die Behältereinrichtung auch als Laugenbehälter bezeichnet. In diesen Laugenbehälter werden die zu waschenden Gegenstände eingebracht. Bei dem Waschvorgang wird dann eine Waschflüssigkeit in Form von mit einem Behandlungsmittel versetzten Wasser in den Laugenbehälter eingebracht.

Das Haushaltsgerät kann lediglich eine Luftaufbereitungsvorrichtung oder eine Mehrzahl von beispielsweise zwei Luftaufbereitungsvorrichtungen umfassen.

Das Gehäuse des Haushaltsgeräts ist vorzugsweise kubisch oder quaderförmig ausgestaltet. Bevorzugt weist das Haushaltsgerät zumindest drei Seitenwandungen auf. Das Gehäuse kann dabei zwei laterale Seitenwandungen umfassen. Ferner kann das Gehäuse eine hintere Seitenwandung umfassen. Schließlich kann das Gehäuse eine vordere Seitenwandung umfassen. Weiterhin ist es von Vorteil, dass das Haushaltsgerät eine Verschlussvorrichtung aufweist, mittels welcher die Behältereinrichtung verschließbar ist. Die Verschlussvorrichtung kann beispielsweise als eine Tür ausgestaltet sein, welche in der vorderen Seitenwandung integriert ist oder anstatt einer vorderen Seitenwandung vorgesehen ist. An der vorderen Seitenwandung und/oder an der Verschlussvorrichtung können gegebenenfalls Bedienelemente für den Nutzer vorgesehen sein. Derartige Bedienelemente sind beispielsweise Programmwahlschalter bei einem Geschirrspüler. Die Luftaufbereitungseinrichtung kann beispielsweise an einer Seitenwandung des Haushaltsgeräts innerhalb oder außerhalb angeordnet sein.

Nach einer vorteilhaften Ausführungsform ist zumindest eine Steuerungseinrichtung vorgesehen. Die zumindest eine Steuerungseinrichtung kann dem Haushaltsgerät oder der Luftaufbereitungsvorrichtung zugeordnet sein. Vorteilhafterweise ist die Steuerungseinrichtung mit der Antriebseinrichtung und/oder der Luftströmungseinrichtung signaltechnisch verbunden. Ferner ist es von Vorteil, dass die Steuerungseinrichtung signaltechnisch mit der Aufbereitungseinrichtung verbunden ist. Es können somit die Strahlungsquelleneinrichtungen durch die Steuerungseinrichtung aktiviert werden. Bevorzugt ist in einem ersten Zustand die Luftaufbereitungsvorrichtung in Form der Aufbereitungseinrichtung und/oder der Luftströmungseinrichtung aktivierbar und in einem zweiten Zustand die Luftaufbereitungsvorrichtung in Form der Aufbereitungseinrichtung und/oder der Luftströmungseinrichtung deaktivierbar.

Bei einer Ausführungsform des Haushaltsgeräts in Form eines Geschirrspülers, einer Waschmaschine oder Ähnlichem könnte vorteilhafterweise in dem zweiten Zustand die Waschflüssigkeit in die Behältereinrichtung beziehungsweise den Laugenbehälter eingebracht werden. Durch die ausreichend ausgelegte Länge der Zuleitungseinrichtung und der Ableitungseinrichtung kann jedoch verhindert werden, dass Waschflüssigkeit in das erste und/oder das zweite Gehäuse gelangt.

Nach einer weiteren vorteilhaften Ausführungsform ist durch die zumindest eine Steuerungseinrichtung ein erstes Zustandssignal bezüglich des ersten Zustands der Luftaufbereitungsvorrichtung empfangbar oder generierbar. Vorzugsweise ist durch die Steuerungseinrichtung ein zweites Zustandssignal bezüglich des zweiten Zustands der Luftaufbereitungsvorrichtung empfangbar oder generierbar.

Nach einer weiteren vorteilhaften Ausführungsform ist eine Eingabeeinrichtung vorgesehen, mittels welcher das erste Zustandssignal und/oder das zweite Zustandssignal generierbar ist. Die Eingabeeinrichtung kann in dem Haushaltsgerät oder in der Luftaufbereitungsvorrichtung implementiert sein.

Vorzugsweise sendet die Eingabeeinrichtung das erste Zustandssignal und/oder das zweite Zustandssignal an die Steuerungseinrichtung. Nach Empfang des ersten Zustandssignals beziehungsweise des zweiten Zustandssignals leitet die Steuerungseinrichtung bevorzugt den ersten oder den zweiten Zustand der Luftaufbereitungsvorrichtung ein. Die Luftaufbereitungsvorrichtung kann somit durch eine entsprechende Eingabe mittels oder über die Eingabeeinrichtung in den ersten Zustand, in welchem die Luftaufbereitungsvorrichtung aktiviert ist, versetzt werden. Weiterhin kann die Luftaufbereitungsvorrichtung somit durch eine entsprechende Eingabe mittels oder über die Eingabeeinrichtung in den zweiten Zustand, in welchem die Luftaufbereitungsvorrichtung deaktiviert ist, versetzt werden. Die Eingabeeinrichtung kann vorzugsweise manuell bedienbar sein. Demnach kann die Eingabeeinrichtung beispielsweise Taster und/oder Schalter und/oder einen Berührbildschirm umfassen. Denkbar wäre auch, dass die Eingabeeinrichtung geeignet ist, Spracheingaben zu verarbeiten. Alternativ oder kumulativ könnte die Eingabeeinrichtung auch eine Gestikerkennung zur Befehlseingabe mittels Gesten umfassen.

Vorzugsweise kann die Eingabeeinrichtung das erste Zustandssignal und/oder das zweite Zustandssignal von einem externen Kommunikationsgerät eines Nutzers empfangen. Vorzugsweise ist die Verbindung zwischen dem externen Kommunikationsgerät und der Eingabeeinrichtung eine drahtlose Verbindung. Ein externes Kommunikationsgerät kann beispielsweise ein Smartphone, ein Tabletcomputer, ein Laptop oder ein ähnliches Gerät sein. Eine entsprechende Drahtlosverbindung kann beispielsweise eine RFID- (radio-frequency identification) Verbindung, eine NFC- (Nahfeldkommunikation) Verbindung, eine WLAN-Verbindung oder eine Mobilfunkverbindung sein. Selbstverständlich können auch noch weitere Drahtlosverbindungen oder auch drahtgebundene Verbindungen zur Anwendung kommen. Demnach kann der Nutzer auf eine sehr bequeme Art und Weise, beispielsweise über das Smartphone, die Luftaufbereitungsvorrichtung aktivieren oder deaktivieren.

Die Aktivierung beziehungsweise die Deaktivierung der Luftaufbereitungsvorrichtung kann jedoch auch vorteilhafterweise automatisch erfolgen. Hierzu generiert vorzugsweise die Steuerungseinrichtung selbst das erste Zustandssignal und/oder das zweite Zustandssignal. Die entsprechenden Zustandssignale werden dann von der Steuerungseinrichtung entsprechend verarbeitet, so dass der erste beziehungsweise der zweite Zustand eingeleitet werden.

Vorzugsweise generiert die zumindest eine Steuerungseinrichtung das erste Zustandssignal und/oder das zweite Zustandssignal anhand von Sensordaten einer Sensoreinrichtung. Vorzugsweise umfasst die Sensoreinrichtung zumindest einen Sensor, welcher den Beladungszustand in der Behältereinrichtung detektiert. Ein solcher Sensor kann beispielsweise ein Gewichtssensor sein, welcher das eingebrachte Gewicht der Gegenstände detektiert. Denkbar wäre auch ein Sensor in Form eines Kamerasystems, welcher einen Beladungszustand erkennen kann, beispielsweise durch Bilderkennung. Es könnte somit vorteilhafterweise bei Detektion einer Beladung, beispielsweise bei einem Geschirrspüler in Form von zu reinigendem Geschirr, der erste Zustand ausgelöst werden, beziehungsweise die Luftaufbereitungsvorrichtung aktiviert werden. Alternativ oder kumulativ umfasst die Sensoreinrichtung vorzugsweise zumindest einen Sensor, welcher bestimmte Gase in der Luft in der Behältereinrichtung detektiert. Derartige Gase können beispielsweise solche sein, welche einen unangenehmen Geruch verursachen. Vorzugsweise könnte bei Detektion eines solchen Gases der erste Zustand ausgelöst werden, beziehungsweise die Luftaufbereitungsvorrichtung aktiviert werden. Nach der Beseitigung des Gases könnte dann der zweite Zustand ausgelöst werden, beziehungsweise die Luftaufbereitungsvorrichtung deaktiviert werden. Vorzugsweise umfasst die Sensoreinrichtung zumindest einen Sensor, welcher eine Öffnung der Verschlussvorrichtung detektiert.

Vorzugsweise kann das zweite Zustandssignal auch bei einem Starten eines bestimmten Programms des Haushaltsgeräts, beispielsweise bei einem Starten eines Waschprogramms erzeugt, beziehungsweise empfangen werden. Dies kann durch die Steuerungseinrichtung erfolgen oder auch durch eine weitere Steuerungseinrichtung.

Nach einer weiteren vorteilhaften Ausführungsform ist eine Zeitgebereinrichtung vorgesehen. Eine solche Zeitgebereinrichtung kann vorzugsweise in der Steuerungseinrichtung integriert sein oder auch als eine weitere Einrichtung in dem Haushaltsgerät vorgesehen sein. Vorteilhafterweise ist das erste Zustandssignal und/oder das zweite Zustandssignal anhand eines vorgegebenen Zeitpunkts oder eines vorgegebenen Zeitintervalls generierbar. Vorteilhafterweise sind somit der Zeitpunkt der Aktivierung des ersten Zustands sowie die Dauer des ersten Zustands vorgebbar.

Vorteilhafterweise umfasst die Steuerungseinrichtung eine Speichereinrichtung, in welcher bestimmte Ablaufprogramme abgelegt sind. Derartige Ablaufprogramme können die sequentielle Ansteuerung bestimmter Einrichtungen, wie die Luftströmungseinrichtung oder die Aufbereitungseinrichtung umfassen. Ebenso kann vorteilhafterweise die Intensität der Ansteuerung dieser Einrichtungen in einem solchen Ablaufprogramm vorgesehen sein. Durch die vorteilhafte Steuerung des Betriebsstromes der Strahlungsquelleneinrichtung kann beispielsweise die photokatalytische Reaktion gesteuert werden. Ebenso kann die Luftstromgeschwindigkeit durch eine vorteilhafte Ansteuerung der Luftströmungseinrichtung gesteuert werden.

Die vorliegende Aufgabe der Erfindung wird auch von einem Verfahren zur Steuerung eines Haushaltsgeräts beziehungsweise einer Luftaufbereitungsvorrichtung gelöst. Das Verfahren kann dabei mit allen bereits obig im Rahmen der Vorrichtung beschriebenen Merkmalen einzeln oder in Kombination miteinander ausgestattet sein und umgekehrt.

Weitere Vorteile, Ziele und Eigenschaften der vorliegenden Erfindung werden anhand nachfolgender Beschreibungen der anliegenden Figuren erläutert. Gleichartige Komponenten können in den verschiedenen Ausführungsformen gleiche Bezugszeichen aufweisen.

In den Figuren zeigen:
- Fig. 1: eine Schnittansicht einer Luftaufbereitungsvorrichtung nach einer Ausführungsform;
- Fig.2: eine Seitenansicht einer Luftaufbereitungsvorrichtung nach einer Ausführungsform;
- Fig.3: eine Seitenansicht einer Luftaufbereitungsvorrichtung nach einer Ausführungsform;
- Fig.4: eine Luftaufbereitungsvorrichtung nach einer Ausführungsform;
- Fig.5: Prinzipskizze einer Aufbereitungseinrichtung nach einer Ausführungsform;
- Fig.6: Prinzipschaltskizze eines Haushaltsgeräts nach einer Ausführungsform Schnittansicht einer Luftaufbereitungsvorrichtung nach einer Ausführungsform;
- Fig.7: Haushaltsgerät umfassend zumindest eine Luftaufbereitungsvorrichtung nach einer Ausführungsform.

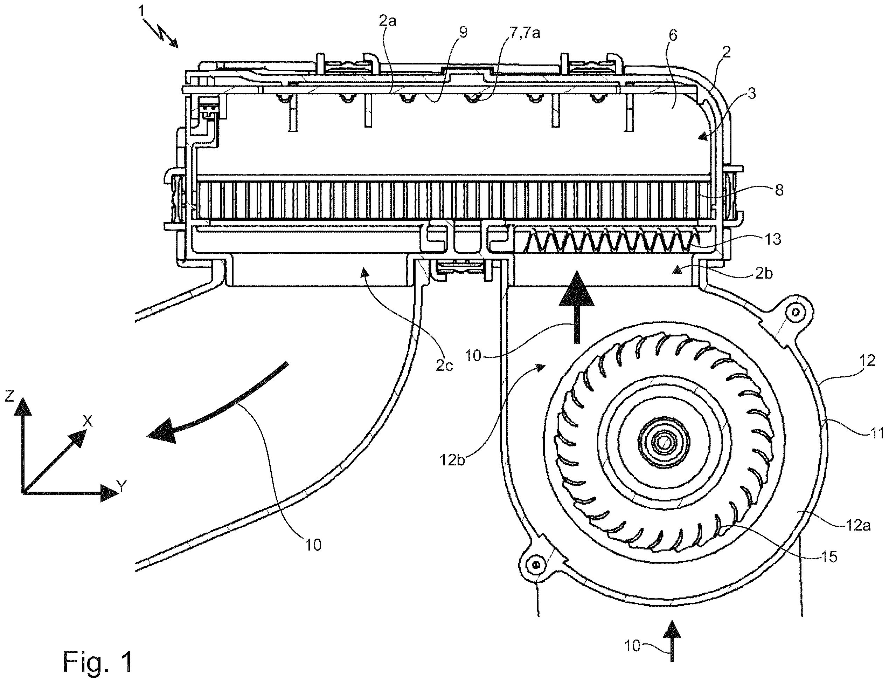

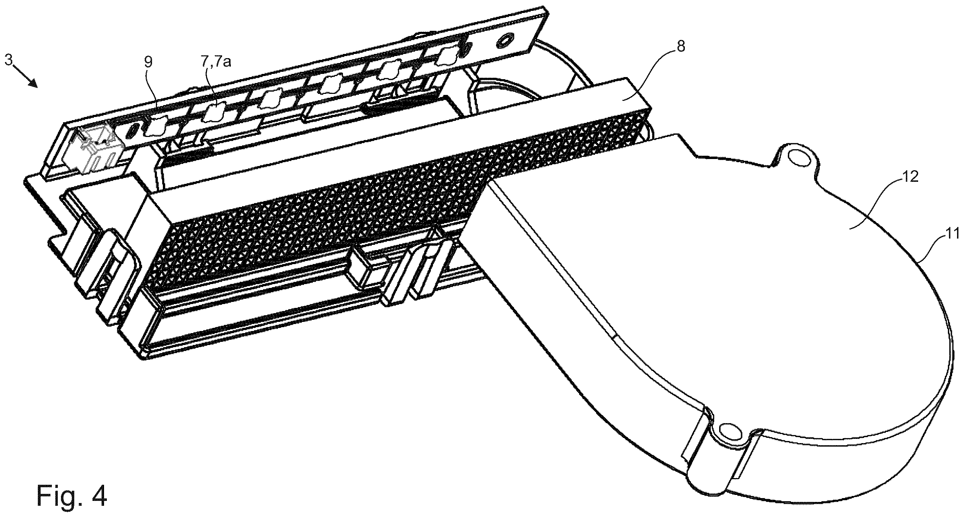

In den Figuren 1 bis 4 wird eine Luftaufbereitungsvorrichtung 1 für ein Haushaltsgerät 100 mit einer Behältereinrichtung 102 gezeigt, wobei die Luftaufbereitungsvorrichtung 1 ein erstes Gehäuse 2 umfasst, in welchem zumindest eine Aufbereitungseinrichtung 3 angeordnet ist, wobei die Luftaufbereitungsvorrichtung 1 eine Zuleitungseinrichtung 4 und eine Ableitungseinrichtung 5 aufweist, wobei die Zuleitungseinrichtung 4 dafür vorgesehen und geeignet ist, einen Luftstrom 10 zu der Aufbereitungseinrichtung 3zu leiten, wobei die Ableitungseinrichtung 5 dafür vorgesehen und geeignet ist, einen Luftstrom 10 von der Aufbereitungseinrichtung 3 weg zu leiten, wobei die zumindest eine Aufbereitungseinrichtung 3 einen Durchtrittskanal 6 für den Luftstrom 10, eine Strahlungsquelleneinrichtung 7 und eine Photokatalyseeinrichtung 8 umfasst.

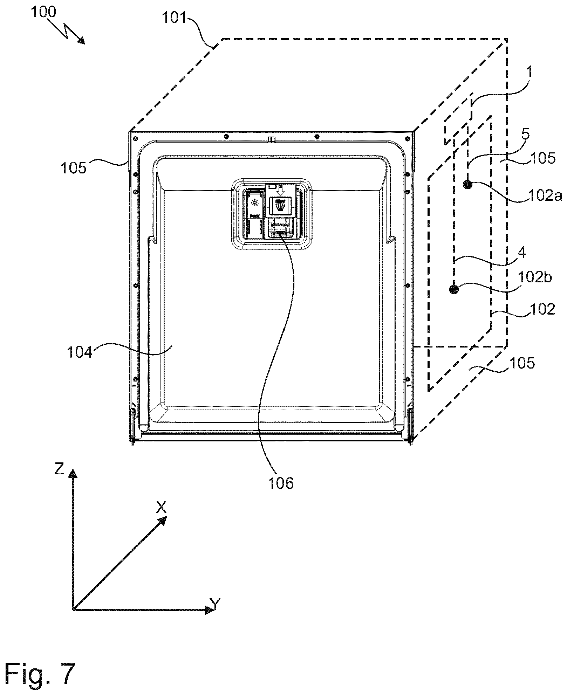

In Figur 7 wird ein Haushaltsgerät 100 umfassend zumindest eine Luftaufbereitungsvorrichtung 1 gezeigt. Das Haushaltsgerät 100 kann ein Geschirrspüler, eine Waschmaschine, ein Kühlschrank oder Ähnliches sein. Das Haushaltsgerät 100 umfasst ein Gehäuse 101 und eine darin vorgesehene Behältereinrichtung 102. Die Luftaufbereitungsvorrichtung 1 ist außerhalb oder innerhalb des Gehäuse 101 des Haushaltsgeräts 100 anordenbar. Ferner ist mittels der zumindest einen Luftströmungseinrichtung 11 ein Luftstrom 10 aus der und/oder in die Behältereinrichtung 102 generierbar. Demnach kann die Luft in der Behältereinrichtung 102 aufbereitetet, gereinigt beziehungsweise desinfiziert werden.

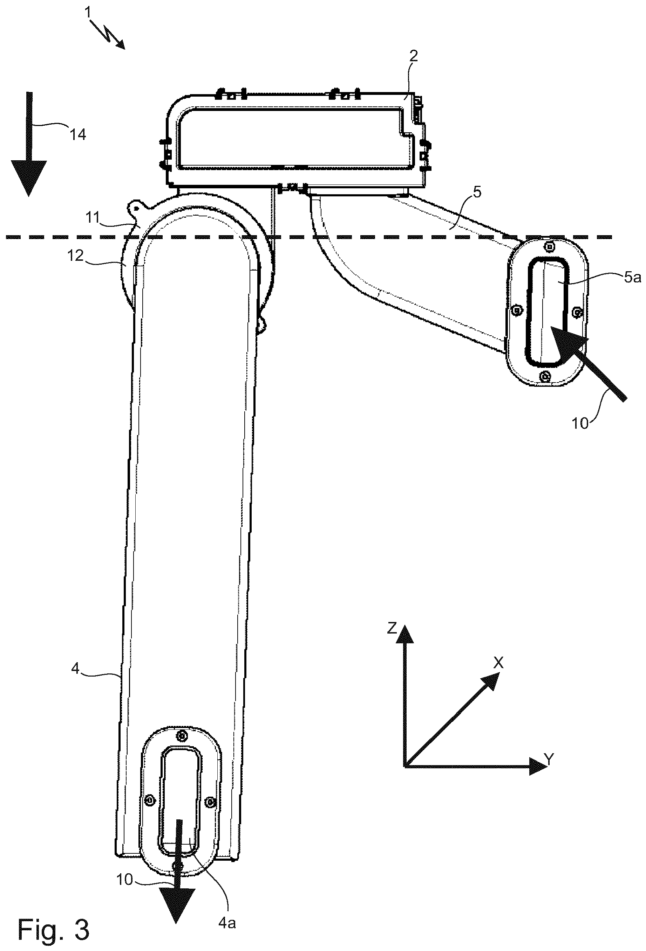

In der Figur 3 ist schematisch eine Aufbereitungseinrichtung 3 dargestellt. Die Aufbereitungseinrichtung 3 erstreckt sich entlang einer Höhenachse Z', einer Breitenachse Y' und einer Längsachse X'. Die Aufbereitungseinrichtung 3 umfasst einen Durchtrittskanal 6 für den Luftstrom 10, eine Strahlungsquelleneinrichtung 7 und eine Photokatalyseeinrichtung 8. Die Strahlungsquelleneinrichtung 7 emittiert elektromagnetische Strahlung. Zur Erzeugung einer photokatalytischen Reaktion ist die Photokatalyseeinrichtung 8 mit zumindest einem Teil der elektromagnetischen Strahlung beaufschlagbar. Die Photokatalyseeinrichtung 8 umfasst eine Photokatalytiseoberfläche 8a, welche zumindest ein photokatalytisches Material umfasst. Das photokatalytische Material ist ein Halbleiter, und umfasst oder ist vorzugsweise Titan(IV)-oxid, TiO2. Die Photokatalytiseoberfläche 8a umfasst dabei Bereiche 8b mit dem photokatalytischen Material. Denkbar sind jedoch auch noch weitere Ausgestaltungen der Photokatalytiseoberfläche 8a. Bei einer Verwendung von Titandioxid ist es vorteilhaft, dass die Strahlungsquelleneinrichtung 7 elektromagnetische Strahlung mit einer Wellenlänge von kleiner als 400 nm, vorzugsweise in einem Bereich von 380 nm bis 315 nm, emittiert. Die Strahlungsquelleneinrichtung 7 umfasst zumindest eine Strahlungsquelle 7a, wobei die zumindest eine Strahlungsquelle 7a eine Leuchtdiode (LED), beziehungsweise ein UV-LED ist. Die Strahlungsquelleneinrichtung 7 umfasst eine Vielzahl von Strahlungsquellen 7a mit einer Gesamtanzahl N_{ges} von Strahlungsquellen 7a. Die zumindest eine Strahlungsquelle 7a ist auf einer Trägereinrichtung 9 angeordnet. Die Trägereinrichtung 9 ist im Wesentlichen plattenartig ausgebildet und im Wesentlichen gegenüberliegend von der Photokatalyseeinrichtung 8 angeordnet. Der Durchtrittskanal 6 für den Luftstrom 10 ist zwischen der Trägereinrichtung 9 und der Photokatalyseeinrichtung 8 vorgesehen. Auf der Trägereinrichtung 9, welche als eine Leiterplatte oder Platine (PCB, printed circuit board) ausgebildet ist, sind weiterhin Treiberbausteine für die Strahlungsquellen beziehungsweise LED's angeordnet.

Die Trägereinrichtung 9 erstreckt sich in einer Ebene, welche durch die Breitenachse Y' und der Längsachse X' aufgespannt wird. Diese Photokatalytiseoberfläche 8a erstreckt sich ebenso in einer Ebene, welche durch die Breitenachse Y' und der Längsachse X' aufgespannt wird. Die Strahlungsquelleneinrichtung 7, beziehungsweise die Trägereinrichtung 9 mit den Strahlungsquellen 7a ist von der Photokatalyseeinrichtung 8, beziehungsweise der Photokatalytiseoberfläche 8a entlang der Höhenachse Z' beabstandet.

Die Luftaufbereitungsvorrichtung 1 erstreckt sich entlang einer Höhenachse Z, einer Längsachse X und einer Breitenachse Y. In den Figuren 1 bis 3 ist ersichtlich, dass das erste Gehäuse 2 quaderförmig ausgestaltet ist. Die vorliegende Erfindung ist jedoch nicht auf eine derartige Ausgestaltung beschränkt. Das erste Gehäuse 2 besteht vorzugsweise aus einem Kunststoff oder einem Metall. In dem ersten Gehäuse 2 ist die Aufbereitungseinrichtung 3 angeordnet. Die Trägereinrichtung 9 ist an einer entlang der Höhenrichtung Z oberen Innenwandung 2a angeordnet. Der Trägereinrichtung 9 gegenüberliegend ist die Photokatalyseeinrichtung 8 angeordnet. Das erste Gehäuse 2 umfasst weiterhin eine Lufteintrittsöffnung 2b und eine Luftaustrittsöffnung 2c. Durch die Lufteintrittsöffnung 2b tritt der Luftstrom 10 in das erste Gehäuse 2 ein. Der Luftstrom 10 gelangt dann vorzugsweise durch Durchgangsöffnungen in der Photokatalyseeinrichtung 8 und/oder in Luftstrompassagen um die Photokatalyseeinrichtung 8 in den Durchtrittskanal 6. Während des Durchtritts durch den Durchgangskanal 6 wird die Luft des Luftstroms 10 aufbereitet. Anschließend gelangt der Luftstrom 10 dann vorzugsweise durch Durchgangsöffnungen in die Photokatalyseeinrichtung 8 und/oder in Luftstrompassagen um die Photokatalyseeinrichtung 8 zu der Luftaustrittsöffnung 2c des ersten Gehäuses 2.

An der Luftaustrittsöffnung 2c des ersten Gehäuses 2 ist die Ableitungseinrichtung 5 angeordnet. Die Ableitungseinrichtung 5 ist dichtend mittels einer kraft- und/oder formschlüssigen Verbindung, beispielsweise einer Schnappverbindung, einer Steckverbindung oder einer Schraubverbindung an dem ersten Gehäuse 2 befestigt. Alternativ ist auch eine stoffschlüssige Verbindung denkbar. Somit besteht eine fluidische Verbindung zwischen der Luftaustrittsöffnung 2c dem ersten Gehäuse 2 und der Ableitungseinrichtung 5, welche den Durchtritt des Luftstroms 10 ermöglicht.

Weiterhin ist eine Luftströmungseinrichtung 11 vorgesehen, welche an dem ersten Gehäuse 2 der Luftaufbereitungsvorrichtung 11 angeordnet ist. Die Luftströmungseinrichtung 11 umfasst ein zweites Gehäuse 12, welches an dem ersten Gehäuse 2 dichtend angeordnet ist. Nach einer alternativen Ausführungsform kann die Luftströmungseinrichtung 11 zumindest teilweise in das erste Gehäuse 2 der Luftaufbereitungsvorrichtung 1 integriert sein. Das zweite Gehäuse 12 besteht vorzugsweise aus einem Kunststoff oder einem Metall. Das zweite Gehäuse 12 umfasst einen im Wesentlichen als ein Kreiszylinder ausgestalteten Hauptabschnitt 12a, an welchem ein Abgabeabschnitt 12b anschließt. Der Abgabeabschnitt 12b des zweiten Gehäuses 12 mündet in dem ersten Gehäuse 2. Die Ableitungseinrichtung 5 ist dichtend mittels einer kraft- und/oder formschlüssigen Verbindung, beispielsweise einer Schnappverbindung, einer Steckverbindung oder einer Schraubverbindung an dem ersten Gehäuse 2 befestigt. Alternativ ist auch eine stoffschlüssige Verbindung denkbar.

Die Luftströmungseinrichtung 11 umfasst einen Rotor 15 beziehungsweise einen Ventilator, welcher in dem Hauptabschnitt 12a mittig angeordnet ist. Durch den Rotor 15 wird der Luftstrom 10 erzeugt. Dementsprechend wird an der Lufteintrittsöffnung 4a beziehungsweise an der Austrittsöffnung 102a der Behältereinrichtung 102 ein Unterdruck erzeugt. Ebenso wird an der Luftaustrittsöffnung 5a und der Eintrittsöffnung 102b der Behältereinrichtung 102 ein Überdruck erzeugt. Der Rotor 15 wird von einer Antriebseinrichtung angetrieben, welche in dem zweiten Gehäuse 12 oder auch außerhalb des zweiten Gehäuses 12 angeordnet sein kann. Die Antriebseinrichtung kann beispielweise ein Elektromotor sein. Zur Kraftübertragung zwischen der Antriebseinrichtung und dem Rotor 15 kann ein Getriebe, eine Welle, eine Spindel oder ein ähnliches geeignetes Kraftübertragungsmittel vorgesehen sein.

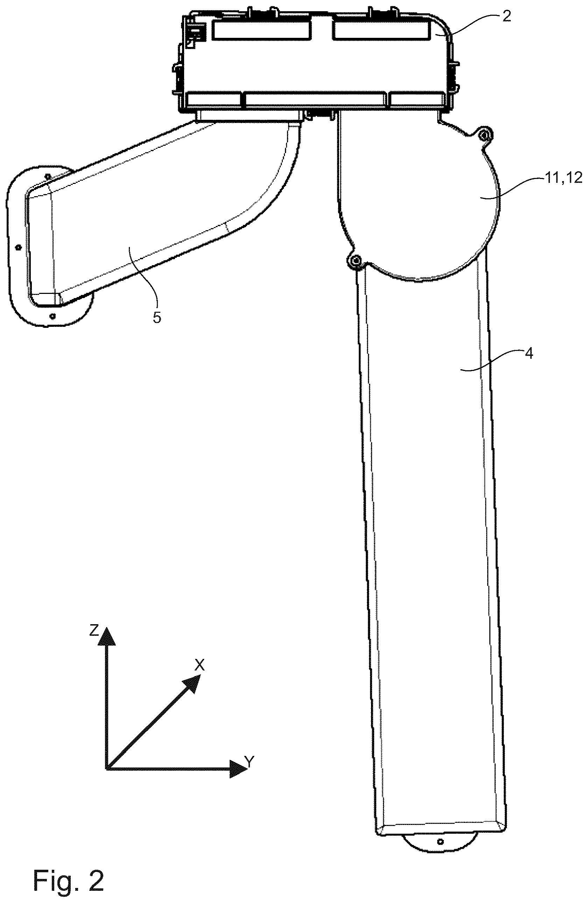

In den Figuren 2 und 3 ist gut ersichtlich, dass die Zuleitungseinrichtung 4 an dem zweiten Gehäuse 12 angeordnet ist. Auch hier kann eine dichtende Verbindung mittels einer kraftund/oder formschlüssigen Verbindung, beispielsweise einer Schnappverbindung, einer Steckverbindung oder einer Schraubverbindung zwischen dem zweiten Gehäuse 12 und der Zuleitungseinrichtung 4 vorhanden sein. Alternativ ist auch hier eine stoffschlüssige Verbindung denkbar. Das zweite Gehäuse 12 ist somit zwischen dem ersten Gehäuse 2 der Luftaufbereitungsvor-richtung 1 und der Zuleitungseinrichtung 4 angeordnet.

Die Zuleitungseinrichtung 4 und/oder die Ableitungseinrichtung 5 sind schlauchartig ausgebildet und bestehen vorteilhafterweise aus einem flexiblen Material. Dies hat den Vorteil, dass eine große Gestaltungsfreiheit hinsichtlich der Anordnung der Luftaufbereitungsvorrichtung in den verschiedensten Haushaltsgeräten besteht. Denkbar wäre selbstverständlich auch eine Ausgestaltung der Zuleitungseinrichtung und/oder der Ableitungseinrichtung 5 in Form von formstabilen Rohren.

Die Zuleitungseinrichtung 4 ist an einer Austrittsöffnung 102a der Behältereinrichtung 102 und die Ableitungseinrichtung 5 ist an einer Eintrittsöffnung 102b der Behältereinrichtung 102 angeordnet. Demnach besteht eine fluidische Verbindung der Lufteintrittsöffnung 4a der Luftaufbereitungsvorrichtung und der Austrittsöffnung 102a der Behältereinrichtung 102 sowie zwischen der Luftaustrittsöffnung 5a der Luftaufbereitungsvorrichtung und der Eintrittsöffnung 102b der Behältereinrichtung 102. In den Figuren 2 und 3 sind Flanschelemente an der Zuleitungseinrichtung 4 und an der Ableitungseinrichtung 5 vorgesehen, mittels welchen die Zuleitungseinrichtung 4 beziehungsweise die Ableitungseinrichtung 5 an der Behältereinrichtung 102 direkt oder indirekt befestigbar sind. Denkbar ist selbstverständlich auch die Verwendung anderweitiger kraft- und/oder formschlüssiger Verbindungen.

Dementsprechend strömt der Luftstrom 10 aus der Behältereinrichtung 102 durch die Austrittsöffnung 102a in die Lufteintrittsöffnung 4a und die Zuleitungseinrichtung 4. Von dort strömt der Luftstrom in das zweite Gehäuse 12 und weiter zu dem ersten Gehäuse 2. Nach dem Durchtreten durch die Aufbereitungseinrichtung strömt der aufbereitete Luftstrom 10 durch die Ableitungseinrichtung 5, die Luftaustrittsöffnung 5a und die Eintrittsöffnung 102b in die Behältereinrichtung 102.

Eine Länge der Zuleitungseinrichtung 4 und/oder der Ableitungseinrichtung 5 sind derart ausgestaltet, dass bei einer Anordnung der Luftaufbereitungsvorrichtung 1 entlang einer Höhenrichtung Z über der Eintrittsöffnung 102b und einer Austrittsöffnung 102a der Behältereinrichtung 102 ein Eintritt von einer Flüssigkeit in das erste Gehäuse 2 weitestgehend unterbunden wird. Dies ist in Figur 7 gut ersichtlich. Durch die Gravitationskraft (dargestellt in Figur 3 durch einen Pfeil 14), welche entlang der Länge oder entlang einem Abschnitt der Länge der Zuleitungseinrichtung 4 und/oder der Ableitungseinrichtung 5 wirkt, kann im Wesentlichen vermieden werden, dass eine Flüssigkeit von der Behältereinrichtung 102 in das erste Gehäuse 2 und/oder das zweite Gehäuse 12 gelangt. Das erste Gehäuse 2 sollte wie in Figur 3 dargestellt über der gestrichelten Linie angeordnet sein. Hierdurch kann vorteilhafterweise eine aufwendige Verschlussmechanik vermieden werden.

Durch den Luftstrom und durch Kondensationsvorgänge kann jedoch trotz allem Flüssigkeit in dem ersten Gehäuse 2 und/oder dem zweiten Gehäuse 12 vorhanden sein. Hierzu umfasst die Luftaufbereitungsvorrichtung 1 eine Entfeuchtungseinrichtung 13, mittels welcher die Feuchtigkeit aus dem ersten 2 und oder zweiten Gehäuse 12 entfernt werden kann. Die Entfeuchtungseinrichtung 13 ist in dem ersten Gehäuse 2 angeordnet. In Figur 1 ist die Entfeuchtungseinrichtung 13 dargestellt, umfassend eine Heizeinrichtung, welche an der Lufteintrittsöffnung 2b vorgesehen ist. Durch die Heizeinrichtung wird die Temperatur erhöht. Die Luft kann demnach mehr Flüssigkeit in Form des Dampfes aufnehmen. Durch den bewegenden Luftstrom wird die Flüssigkeit aus dem Gehäuse transportiert.

In Figur 7 ist ein Haushaltsgerät 100 dargestellt. Das Gehäuse 101 des Haushaltsgeräts 100 kann eine Verschlussvorrichtung 104, beispielsweise eine Tür umfassen, mittels welcher die Behältereinrichtung 102 verschließbar ist. Es kann dabei zumindest eine Luftaufbereitungsvorrichtung 1 in oder an der Verschlussvorrichtung 104 angeordnet sein. Das Haushaltsgerät 100 kann im Wesentlichen kubisch oder quaderförmig ausgebildet sein und zwei laterale Seitenwandungen 105a und eine hintere Seitenwandung 105b oder auch eine Rückwand, welche vorzugsweise der Verschlussvorrichtung 104 gegenüberliegt, umfassen. Schließlich kann das Gehäuse eine vordere Seitenwandung 105 umfassen. Die Verschlussvorrichtung 104 kann beispielsweise als eine Tür ausgestaltet sein, welche in der vorderen Seitenwandung 105 integriert ist oder anstatt einer vorderen Seitenwandung 105 vorgesehen ist. An der vorderen Seitenwandung und/oder an der Verschlussvorrichtung 104 können gegebenenfalls Bedienelemente für den Nutzer vorgesehen sein. Derartige Bedienelemente sind beispielsweise Programmwahlschalter bei einem Geschirrspüler. Die Luftaufbereitungsvorrichtung kann einer beliebigen Seitenwandung innerhalb oder außerhalb des Gehäuses angeordnet sein.

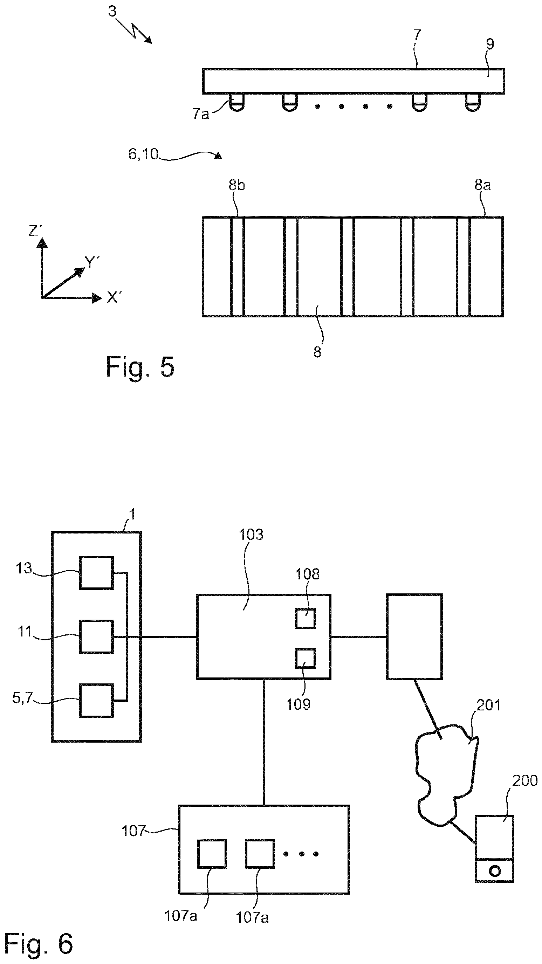

In Figur 6 ist eine Prinzipschaltskizze für ein Haushaltsgerät 100 mit zumindest einer Luftaufbereitungsvorrichtung 1 dargestellt. Demnach ist eine Steuerungseinrichtung 103 vorgesehen, welche dem Haushaltsgerät oder der Luftaufbereitungsvorrichtung 1 zuordenbar ist. Die Steuerungseinrichtung 103 ist mit der Luftaufbereitungsvorrichtung 1 signaltechnisch verbunden. Es können somit die Aufbereitungseinrichtung 5, insbesondere der Strahlungsquelleneinrichtung 7, die Luftströmungseinrichtung 11, insbesondere die Antriebseinrichtung für den Rotor 15 und die Entfeuchtungseinrichtung 13 mit der Steuerungseinrichtung 103 angesteuert werden.

Nach einer weiteren Ausführungsform umfasst die Luftaufbereitungsvorrichtung 1 selbst eine Steuerungseinrichtung, welche mit einer Steuerungseinrichtung des Haushaltsgeräts verbunden ist oder auch autark von dieser Steuerungseinrichtung ist. Demnach würde eine Eingabeeinrichtung mit der Steuerungseinrichtung der Luftaufbereitungsvorrichtung 1 kommunizieren.

Durch die Steuerungseinrichtung 103 ist ein erstes Zustandssignal bezüglich des ersten Zustands der Luftaufbereitungsvorrichtung 1 empfangbar oder generierbar. Ferner ist durch die Steuerungseinrichtung 103 ein zweites Zustandssignal bezüglich des zweiten Zustands der Luftaufbereitungsvorrichtung 1 empfangbar oder generierbar. In dem ersten Zustand ist die Luftaufbereitungsvorrichtung 1 aktiviert und in einem zweiten Zustand ist die Luftaufbereitungsvorrichtung 1 deaktiviert. In einem aktivierten Zustand der Luftaufbereitungsvorrichtung 1 sind vorteilhafterweise die Aufbereitungseinrichtung 6 beziehungsweise die Strahlungsquelleneinrichtung 7 und die Luftströmungseinrichtung 11 aktiviert.

Bei einer Ausführungsform des Haushaltsgeräts 100 in Form eines Geschirrspülers, einer Waschmaschine oder Ähnlichem wird in dem zweiten Zustand die Waschflüssigkeit in die Behältereinrichtung 102 beziehungsweise den Laugenbehälter eingebracht.

Die Steuerungseinrichtung 103 ist signaltechnisch mit zumindest einer Eingabeeinrichtung 106 verbunden, mittels welcher das erste Zustandssignal und/oder das zweite Zustandssignal generierbar ist. Die Eingabeeinrichtung 106 sendet das erste Zustandssignal und/oder das zweite Zustandssignal an die zumindest eine Steuerungseinrichtung 103, woraufhin diese den ersten oder den zweiten Zustand der Luftaufbereitungsvorrichtung 1 einleitet. Die Luftaufbereitungsvorrichtung 1 kann somit durch eine entsprechende Eingabe mittels oder über die Eingabeeinrichtung 106 in den ersten Zustand, in welchem die Luftaufbereitungsvorrichtung 1 aktiviert ist, versetzt werden. Weiterhin kann die Luftaufbereitungsvorrichtung 1 durch eine entsprechende Eingabe mittels oder über die Eingabeeinrichtung 106 in den zweiten Zustand, in welchem die Luftaufbereitungsvorrichtung 1 deaktiviert ist, versetzt werden. Die Eingabeeinrichtung 106 kann vorzugsweise manuell bedienbar sein. Demnach kann die Eingabeeinrichtung 106 beispielsweise Taster und/oder Schalter und/oder einen Berührbildschirm umfassen. Vorzugsweise kann die Eingabeeinrichtung 106 das erste Zustandssignal und/oder das zweite Zustandssignal von einem externen Kommunikationsgerät 200 eines Nutzers empfangen. Vorzugsweise ist die Verbindung zwischen dem externen Kommunikationsgerät und der Eingabeeinrichtung eine drahtlose Verbindung 201. Ein externes Kommunikationsgerät 200 kann beispielsweise ein Smartphone, ein Tabletcomputer, ein Laptop oder ein ähnliches Gerät sein. Eine entsprechende Drahtlosverbindung 201 kann beispielsweise eine RFID- (radio-frequency identification) Verbindung, eine NFC- (Nahfeldkommunikation) Verbindung, eine WLAN-Verbindung oder eine Mobilfunkverbindung sein. Selbstverständlich können auch noch weitere Drahtlosverbindungen oder auch drahtgebundene Verbindungen zur Anwendung kommen.

Die Aktivierung beziehungsweise die Deaktivierung der Luftaufbereitungsvorrichtung 1 kann jedoch auch vorteilhafterweise automatisch erfolgen. Hierzu generiert vorzugsweise die Steuerungseinrichtung 103 selbst das erste Zustandssignal und/oder das zweite Zustandssignal. Die entsprechenden Zustandssignale werden dann von der Steuerungseinrichtung 103 entsprechend verarbeitet, so dass der erste beziehungsweise der zweite Zustand eingeleitet werden. Vorzugsweise generiert die zumindest eine Steuerungseinrichtung 103 das erste Zustandssignal und/oder das zweite Zustandssignal anhand von Sensordaten einer Sensoreinrichtung 107. Die Sensoreinrichtung 107 kann zumindest einen Sensor 107a umfassen, welcher den Beladungszustand in der Behältereinrichtung 102 detektiert. Ein solcher Sensor 107a kann beispielsweise ein Gewichtssensor sein, welcher das eingebrachte Gewicht der Gegenstände detektiert. Denkbar wäre auch ein Sensor 107a in Form eines Kamerasystems, welcher einen Beladungszustand erkennen kann, beispielsweise durch Bilderkennung. Es könnte somit vorteilhafterweise bei Detektion einer Beladung, beispielsweise bei einem Geschirrspüler in Form von zu reinigendem Geschirr, der erste Zustand ausgelöst werden, beziehungsweise die Luftaufbereitungsvorrichtung 1 aktiviert werden. Alternativ oder kumulativ umfasst die Sensoreinrichtung 107 zumindest einen Sensor 107a, welcher bestimmte Gase in der Luft in der Behältereinrichtung 102 detektiert. Derartige Gase können beispielsweise solche sein, welche einen unangenehmen Geruch verursachen. Vorzugsweise könnte bei Detektion eines solchen Gases der erste Zustand ausgelöst werden, beziehungsweise die Luftaufbereitungsvorrichtung 1 aktiviert werden. Nach der Beseitigung des Gases könnte dann der zweite Zustand ausgelöst werden, beziehungsweise die Luftaufbereitungsvorrichtung 1 deaktiviert werden. Ferner könnte die Sensoreinrichtung 107 zumindest einen Sensor 107a umfassen, welcher eine Öffnung der Verschlussvorrichtung 104 des Haushaltsgeräts 100 detektiert. Somit könnte bei einer Öffnung der Verschlussvorrichtung 104 der zweite Zustand ausgelöst werden, beziehungsweise die Luftaufbereitungsvorrichtung 1 deaktiviert werden. Nach dem Schließen der Verschlussvorrichtung 104 könnte der erste Zustand ausgelöst werden, beziehungsweise die Luftaufbereitungsvorrichtung 1 aktiviert werden.

Nach einer weiteren vorteilhaften Ausführungsform ist eine Zeitgebereinrichtung 108 vorgesehen. Eine solche Zeitgebereinrichtung 108 kann vorzugsweise in der Steuerungseinrichtung 103 integriert sein oder auch als eine weitere Einrichtung in dem Haushaltsgerät 100 vorgesehen sein. Das erste Zustandssignal und/oder das zweite Zustandssignal können anhand eines vorgegebenen Zeitpunkts oder eines vorgegebenen Zeitintervalls generiert werden. Somit ist der Zeitpunkt der Aktivierung des ersten Zustands sowie die Dauer des ersten Zustands vorgebbar.

Die Steuerungseinrichtung 103 umfasst eine Speichereinrichtung 109, in welcher bestimmte Ablaufprogramme abgelegt sind. Derartige Ablaufprogramme können die sequentielle Ansteuerung bestimmter Einrichtungen wie die Luftströmungseinrichtung 11 oder die Aufbereitungseinrichtung 3 oder auch die Intensität der Ansteuerung dieser Einrichtungen umfassen.

Bei bisherigen Geschirrspülern wird beispielsweise die Desinfektion durch ein entsprechendes Waschprogramm durchgeführt. Eine entsprechende Luftreinigung beziehungsweise Desinfektion der Luft in dem Laugenbehälter, wie in der vorliegenden Erfindung, ist bisher nicht vorgesehen. In einem Geschirrspüler kann beispielsweise die vorliegende Luftaufbereitungsvorrichtung auch in einem zusätzlichen Durchgang in dem Trocknungsprogramm implementiert werden.

Zusammenfassend bietet die vorliegende Luftaufbereitungsvorrichtung eine maximale Kosteneffizienz. Durch die Unterbringung der Aufbereitungseinrichtung und somit der essentiellen Komponenten der Luftaufbereitungsvorrichtung in einem Gehäuse, auch genannt eine "common core box" und die weitere Verwendung äußerst flexibel gestaltbarer Zuleitungs- beziehungsweise Ableitungseinrichtungen werden eine Vielzahl von Implementierungsmöglichkeiten für Haushaltsgeräten, beispielsweise Geschirrspülern (Einlass- und Auslasspositionierung) geschaffen. Durch die bisher spezielle Ausgestaltung von Luftaufbereitungsvorrichtungen in Haushaltsgeräten, gab es keine Möglichkeit der Maximierung der Verwendbarkeit von gleichartigen Komponenten, vor allem der von kostentreibenden Komponenten. In dem Gehäuse beziehungsweise der "Common Core Box" werden alle kostentreibenden Komponenten wie UV-LED, Treiber und Photokatalysator untergebracht, so dass ein modular verwendbare Luftaufbereitungsvorrichtung bereitgestellt wird, welche in einer Vielzahl von Haushaltsgeräten einsetzbar ist. Die kompakte Bauweise erlaubt die Implementierung an jeder verfügbaren Stelle im Gerät.

Die Anmelderin behält sich vor, sämtliche in den Anmeldungsunterlagen offenbarten Merkmale als erfindungswesentlich zu beanspruchen, sofern sie einzeln oder in Kombination gegenüber dem Stand der Technik neu sind. Es wird weiterhin darauf hingewiesen, dass in den einzelnen Figuren auch Merkmale beschrieben wurden, welche für sich genommen vorteilhaft sein können. Der Fachmann erkennt unmittelbar, dass ein bestimmtes in einer Figur beschriebenes Merkmal auch ohne die Übernahme weiterer Merkmale aus dieser Figur vorteilhaft sein kann. Ferner erkennt der Fachmann, dass sich auch Vorteile durch eine Kombination mehrerer, in einzelnen oder in unterschiedlichen Figuren gezeigter Merkmale ergeben können.

### Bezugszeichenliste

- 1: Luftaufbereitungsvorrichtung
- 2: erstes Gehäuse
- 2a: obere Innenwandung
- 2b: Lufteintrittsöffnung
- 2c: Luftaustrittsöffnung
- 3: Aufbereitungseinrichtung
- 4: Zuleitungseinrichtung
- 4a: Lufteintrittsöffnung
- 5: Ableitungseinrichtung
- 5a: Luftaustrittsöffnung
- 6: Durchtrittskanal
- 7: Strahlungsquelleneinrichtung
- 7a: Strahlungsquelle
- 8: Photokatalyseeinrichtung
- 8a: Photokatalytiseoberfläche
- 8b: Bereiche der Photokatalytiseoberfläche mit dem photokatalytischen Material
- 9: Trägereinrichtung
- 10: Luftstrom
- 11: Luftströmungseinrichtung
- 12: zweites Gehäuse
- 12a: Hauptabschnitt
- 12b: Abgabeabschnitt
- 13: Entfeuchtungseinrichtung
- 14: Pfeil
- 15: Rotor
- 100: Haushaltsgerät
- 101: Gehäuse
- 102: Behältereinrichtung
- 102a: Austrittsöffnung
- 102b: Eintrittsöffnung
- 103: Steuerungseinrichtung
- 104: Verschlussvorrichtung
- 105: Seitenwandung
- 105a: laterale Seitenwandung
- 105b: hintere Seitenwandung
- 106: Eingabeeinrichtung
- 107: Sensoreinrichtung
- 107a: Sensor
- 108: Zeitgebereinrichtung
- 109: Speichereinrichtung
- 200: Kommunikationsgerät
- 201: Drahtlosverbindung
- X: Längsachse der Luftaufbereitungsvorrichtung
- Y: Breitenachse der Luftaufbereitungsvorrichtung
- Z: Höhenachse der Luftaufbereitungsvorrichtung
- X': Längsachse der Aufbereitungseinrichtung
- Y': Breitenachse der Aufbereitungseinrichtung
- Z': Höhenachse der Aufbereitungseinrichtung

## Patentansprüche

1. Luftaufbereitungsvorrichtung (1) für ein Haushaltsgerät (100) mit einer Behältereinrichtung (102),
**dadurch gekennzeichnet, dass**
die Luftaufbereitungsvorrichtung (1) ein erstes Gehäuse (2) umfasst, in welchem zumindest eine Aufbereitungseinrichtung (3) angeordnet ist, wobei die Luftaufbereitungsvorrichtung (1) eine Zuleitungseinrichtung (4) und eine Ableitungseinrichtung (5) aufweist, wobei die Zuleitungseinrichtung (4) dafür vorgesehen und geeignet ist, einen Luftstrom (10) zu der Aufbereitungseinrichtung (3) zu leiten, wobei die Ableitungseinrichtung (5) dafür vorgesehen und geeignet ist, einen Luftstrom (10) von der Aufbereitungseinrichtung (3) weg zu leiten, wobei die zumindest eine Aufbereitungseinrichtung (3) einen Durchtrittskanal (6) für den Luftstrom (10), eine Strahlungsquelleneinrichtung (7) und eine Photokatalyseeinrichtung (8) umfasst.

2. Luftaufbereitungsvorrichtung (1) nach Anspruch 1,
**dadurch gekennzeichnet, dass**
die Strahlungsquelleneinrichtung (7) elektromagnetische Strahlung emittiert, wobei zur Erzeugung einer photokatalytischen Reaktion die Photokatalyseeinrichtung (8) mit zumindest einem Teil der elektromagnetischen Strahlung beaufschlagbar ist, wobei die Photokatalyseeinrichtung (8) eine Photokatalytiseoberfläche (8a) umfasst, welche zumindest ein photokatalytisches Material umfasst, wobei das photokatalytische Material ein Halbleiter ist, wobei das photokatalytische Material Titan(IV)-oxid, TiO₂ umfasst.

3. Luftaufbereitungsvorrichtung (1) nach Anspruch 2,
**dadurch gekennzeichnet, dass**
die Strahlungsquelleneinrichtung (7) zumindest eine Strahlungsquelle (7a) umfasst, wobei die zumindest eine Strahlungsquelle (7a) eine Leuchtdiode (LED) ist, wobei die Strahlungsquelleneinrichtung (7) elektromagnetischen Strahlung mit einer Wellenlänge von kleiner als 400 nm emittiert, wobei die Strahlungsquelleneinrichtung (7) elektromagnetische Strahlung mit einer Wellenlänge in einem Bereich von 380 nm bis 315 nm emittiert.

4. Luftaufbereitungsvorrichtung (1) nach Anspruch 3,
**dadurch gekennzeichnet, dass**
die zumindest eine Strahlungsquelle (7a) auf einer Trägereinrichtung (9) angeordnet ist, wobei die Trägereinrichtung (9) plattenartig ausgebildet ist, wobei die Tragereinrichtung (9) im Wesentlichen gegenüberliegend von der Photokatalyseeinrichtung (8) angeordnet ist, wobei der Durchtrittskanal (6) für den Luftstrom (10) zwischen der Trägereinrichtung (9) und der Photokatalyseeinrichtung (8) vorgesehen ist.

5. Luftaufbereitungsvorrichtung (1) nach einem der vorhergehenden Ansprüche,
**dadurch gekennzeichnet, dass**
zumindest eine Luftströmungseinrichtung (11) vorgesehen ist, mittels welcher der Luftstrom (10) in und/oder aus der Luftaufbereitungsvorrichtung (1) generierbar ist, wobei die Zuleitungseinrichtung (4) eine Lufteintrittsöffnung (4a) aufweist und die Aufbereitungseinrichtung (3) eine Luftaustrittsöffnung (5a) aufweist, wobei die zumindest eine Luftströmungseinrichtung (11) an der zumindest einen Lufteintrittsöffnung (4a) einen Unterdruck erzeugt, wobei durch diesen Unterdruck der Luftstrom (10) in die zumindest eine Lufteintrittsöffnung (4a) erzeugbar ist.

6. Luftaufbereitungsvorrichtung (1) nach Anspruch 5,
**dadurch gekennzeichnet, dass**
die Luftströmungseinrichtung (11) zumindest teilweise in das erste Gehäuse (2) der Luftaufbereitungsvorrichtung (1) integriert ist.

7. Luftaufbereitungsvorrichtung (1) nach Anspruch 5
**dadurch gekennzeichnet, dass**
die Luftströmungseinrichtung (11) an dem ersten Gehäuse (2) der Luftaufbereitungsvorrichtung (11) angeordnet ist, wobei die Luftströmungseinrichtung (11) ein zweites Gehäuse (12) umfasst, welches an dem ersten Gehäuse (2) dichtend angeordnet ist, wobei das zweite Gehäuse (12) zwischen dem ersten Gehäuse (2) der Luftaufbereitungsvorrichtung (1) und der Zuleitungseinrichtung (4) angeordnet ist.

8. Luftaufbereitungsvorrichtung (1) nach einem der vorhergehenden Ansprüche,
**dadurch gekennzeichnet, dass**
die Zuleitungseinrichtung (4) und/oder die Ableitungseinrichtung (5) schlauchartig ausgebildet sind, wobei die Zuleitungseinrichtung (4) und/oder die Ableitungseinrichtung (5) aus einem flexiblen Material hergestellt sind, wobei eine Länge der Zuleitungseinrichtung (4) und/oder der Ableitungseinrichtung (5) derart ausgestaltet ist, dass bei einer Anordnung der Luftaufbereitungsvorrichtung (1) entlang einer Höhenrichtung (Z) über einer Eintrittsöffnung und einer Austrittsöffnung der Behältereinrichtung (102) ein Eintritt von einer Flüssigkeit in das erste Gehäuse (2) weitestgehend unterbunden wird.

9. Luftaufbereitungsvorrichtung (1) nach einem der vorhergehenden Ansprüche,
**dadurch gekennzeichnet, dass**
Luftaufbereitungsvorrichtung (1) eine Entfeuchtungseinrichtung (13) umfasst, wobei die Entfeuchtungseinrichtung (13) in dem ersten Gehäuse (2) angeordnet ist, wobei die Entfeuchtungseinrichtung (13) eine Heizeinrichtung umfasst.

10. Haushaltsgerät (100) umfassend zumindest eine Luftaufbereitungsvorrichtung (1) nach einem der vorhergehenden Ansprüche 1 bis 9, wobei das Haushaltsgerät (100) ein Gehäuse (101) und eine darin vorgesehene Behältereinrichtung (102) umfasst.

11. Haushaltsgerät (100) nach Anspruch 9,
**dadurch gekennzeichnet, dass**
zumindest eine Steuerungseinrichtung (103) vorgesehen ist, welche die Komponenten der Luftaufbereitungsvorrichtung ansteuert.

12. Haushaltsgerät (100) nach Anspruch 11,
**dadurch gekennzeichnet, dass**
durch die Steuerungseinrichtung (103) ein erstes Zustandssignal bezüglich des ersten Zustands der Luftaufbereitungsvorrichtung (1) empfangbar oder generierbar ist, wobei durch die Steuerungseinrichtung (103) ein zweites Zustandssignal bezüglich des zweiten Zustands der Luftaufbereitungsvorrichtung (1) empfangbar oder generierbar ist, wobei in dem ersten Zustand die Luftaufbereitungsvorrichtung (1) aktivierbar und in dem zweiten Zustand die Luftaufbereitungsvorrichtung 1 deaktivierbar ist.

13. Haushaltsgerät (100) nach Anspruch 12,
**dadurch gekennzeichnet, dass**
zumindest eine Eingabeeinrichtung (106) vorgesehen ist mittels welcher das erste Zustandssignal und/oder das zweite Zustandssignal generierbar ist, wobei die Eingabeeinrichtung (106) das erste Zustandssignal und/oder das zweite Zustandssignal an die zumindest eine Steuerungseinrichtung (103) sendet, worauf hin diese den ersten oder den zweiten Zustand der Luftaufbereitungsvorrichtung (1) einleitet, wobei die Eingabeeinrichtung (106) das erste Zustandssignal und/oder das zweite Zustandssignal von einem externen Kommunikationsgerät (200) eines Nutzers empfangen kann.

14. Haushaltsgerät (100) nach Anspruch 12 oder 13,
**dadurch gekennzeichnet, dass**
die zumindest eine Steuerungseinrichtung (103) das erste Zustandssignal und/oder das zweite Zustandssignal anhand von Sensordaten einer Sensoreinrichtung (107) generiert, wobei die Sensoreinrichtung zumindest einen Sensor (107a) umfasst, welcher den Beladungszustand in der Behältereinrichtung detektiert, wobei die Sensoreinrichtung (107) zumindest einen Sensor (107a) umfasst, welcher bestimmte Gase in der Luft in der Behältereinrichtung detektiert, wobei die Sensoreinrichtung (107) zumindest einen Sensor (107a) umfasst, welcher eine Öffnung der Verschlussvorrichtung detektiert.

15. Haushaltsgerät (100) nach einem der Ansprüche 12 bis 14,
**dadurch gekennzeichnet, dass**
eine Zeitgebereinrichtung (108) vorgesehen ist, wobei das erste Zustandssignal und/oder das zweite Zustandssignal anhand eines vorgegebenen Zeitpunkts oder eines vorgegebenen Zeitintervalls generierbar ist.
